# EUROPEAN PATENT SPECIFICATION

(11) **EP 0 974 331 B1**
(45) Date of publication and mention of the grant of the patent: **19.05.2004**
(21) Application number: 99202386.1
(22) Date of filing: 20.07.1999
(51) Int. Cl.: A61J 1/10, B65D 81/32, B65D 25/08

(54) **Container for haemofiltration solutions**
Beutel für Hämofiltrationsflüssigkeit
Poche pour solutions d'hémofiltration

(30) Priority: 23.07.1998 IT MI980509 U
(43) Date of publication of application: 26.01.2000
(73) Proprietor: Bluehill Trust Reg, 9490 Vaduz (LI)
(72) Inventor: Valoti, Michele, 6900 Massagno (CH)
(74) Representative: Mittler, Enrico

(56) References cited:
- EP-A- 0 442 406
- WO-A-95/08299
- US-A- 4 403 992

## Description

The present invention refers to a container for haemofiltration solutions and for liquids for medical use in general, in particular with two distinct compartments or pockets, each containing substances that can be mixed together at a determined moment and through a particular connection.

Haemofiltration is a process in which a gradient of hydrostatic pressure transfers the water and the solutes of the blood through a membrane by convection (ultrafiltration). It sets an alternative to the traditional dialysis, that is instead based on the principle of the diffusion, as a mean to purify the blood of the uraemic patients.

Haemofiltration and the various types of membranes that allow its execution are created with the object of assuring a better removal of all those toxic solutes that are removed in minimum part with diffusion, mainly for problems connected with the dimensions of the molecules.

During dialysis, the ultrafiltered matter with the nitrogenous wastes is eliminated and it is replaced by an equal volume of sterile and apirogenous substitution liquid, having the same electrolythic composition as the extracellular liquid but that is, obviously, free of nitrogenous wastes.

The hydroelectrolytic reintegrating solution for haemofiltration is usually made up of several substances (for instance HCO₃⁻ ions and Ca⁺⁺ and Mg⁺⁺ ions) that are incompatible with each other in that they can cause chemical precipitation if in contact inside a single pocket.

In order to obviate all that, the incompatible substances (that still with reference to the previous example are two, that is one comprising HCO₃⁻ ions and the other comprising Ca⁺⁺ and Mg⁺⁺ ions) are inserted in two separate pockets or compartments, usually having different dimensions, that are put into communication only subsequently.

Separate pockets or compartments, usually having different directions, that are put into communication only subsequently.

In particular, the content of the smaller pocket is introduced into the larger pocket only at the moment of use, when the mixture of the two components is to be administered to the patient under dialysis, and the flow of the solution must occur in a single possible direction.

EP-A-0 442 406 discloses a mixing container for plural substances defined in the preamble of claim 1 including.

In view of all that, object of the present invention has been to provide a pocket container for haemofiltration solutions, made up of two separate compartments or pockets, each one containing substances that can be mixed together only at a determined moment and through a particular connection between the two aforesaid pockets.

According to the present invention such object is attained with a pocket container for haemofiltration solutions and for liquids for medical use in general, as defined in claim 1.

The characteristics of the present invention will be rendered more evident by the following detailed description of an embodiment thereof, that is illustrated as a non limiting example in the enclosed drawings in which:
Figure 1 represents a pocket container with two compartments and a connection between them, according to the present invention;
Figure 2 shows a magnified section of one part of the container of figure 1, in particular of a connection line between the two compartments, at an operating stage;
Figures 3 and 4 show a magnified section of another part of the container of Figure 1, in particular of a closing element of the connection line, at two different operating stages;
Figure 5 shows the same section of figure 2, at a different operating stage.

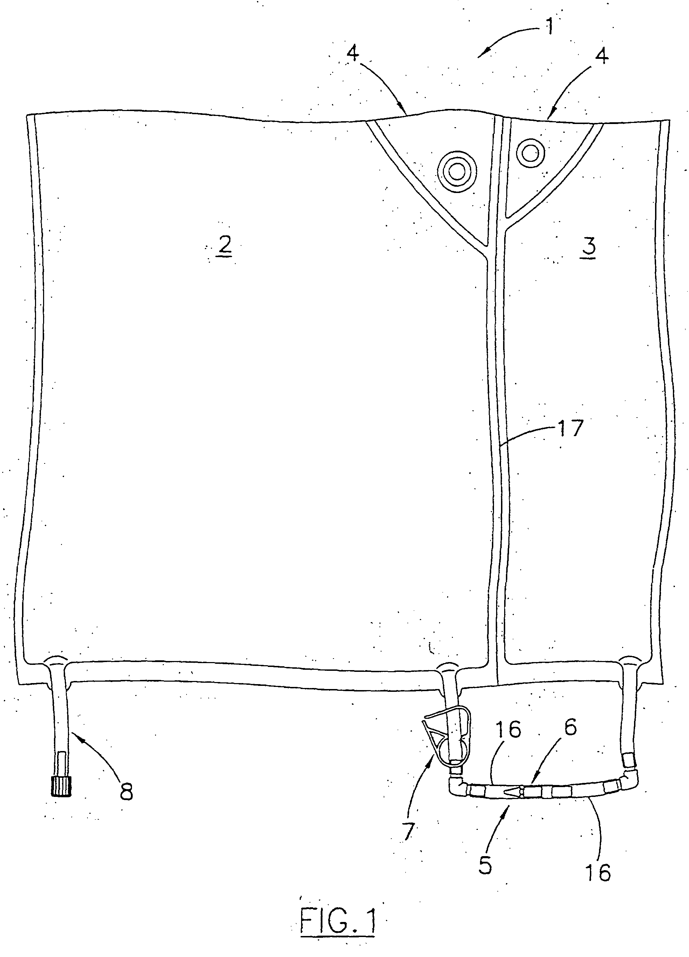

With reference to Figure 1, there is shown a pocket container 1 comprising two separate compartments 2 and 3 (they can also be two separate pockets) that are joined to each other along a frangible line 17, each one with its own coupling element 4.

The two compartments 2 and 3 are coupled by means of a connection line 5 made up of a number hoses 16 that are connected with each other, and comprising a frangible stopper 6 thereinside. A closing member 7 is provided on the connection line 5.

The larger pocket 2 is further provided with a hose 8 that is suitable for the connection between the same pocket and the patient under dialysis.

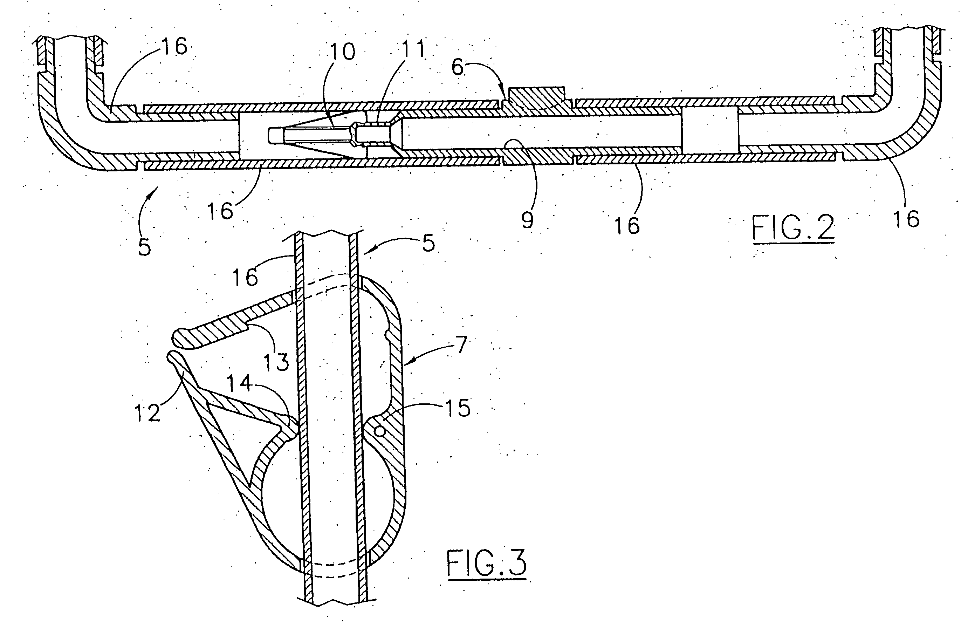

In Figures 2 and 5 a part of the connection line 5 between the two compartments 2 and 3 is shown in magnified section, at two different operating stages. In particular, it is possible to observe the frangible stopper 6 inserted in the connection line 5 in order to join the two parts thereof. The frangible stopper 6 comprises a hollow cylindrical part 9 in which a solution can flow, and a closed end 10, provided with tabs, which prevents such flow (Figure 2). The two parts 9 and 10 are coupled through a frangible hollow part 11.

By exerting a pressure from the outside on the connection line 5 and in particular on the part 11 of the frangible stopper 6 that couples the cylindrical part 9 with the tabbed end 10, the breaking of the same stopper is caused, with the consequence of creating a connection between the two compartments 2 and 3 and allowing to manually force (by simple pressure on the compartment 3 or by separating and completely or partially rolling up the same compartment 3 along the frangible line 17) a flow inside the connection line 5 (Figure 5). Such flow is not slowed by the tabbed end 10 in that the same tabs allow the passage of the solution in the space found between them.

The operation of breaking of the frangible stopper 6 is carried out at the moment of use, when one wants to put the two compartments 2 and 3 into communication in order to cause the solution contained in the pocket 3 to flow inside the pocket 2 and therefore to administer the mixture of the two substances to the patient through the hose 8.

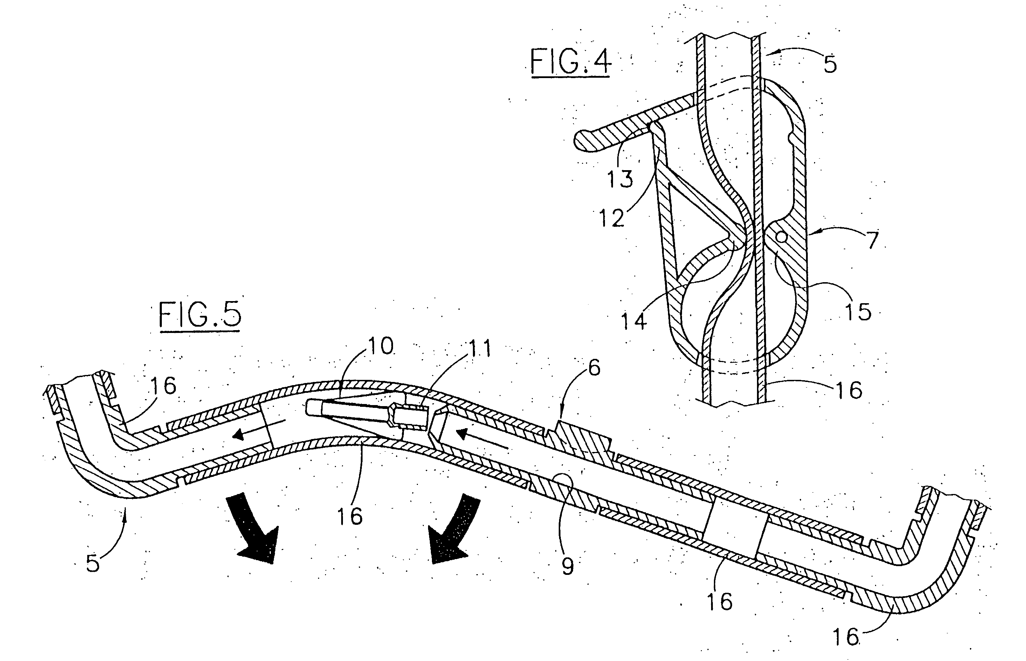

In Figures 3 and 4 a magnified section of the closing member 7 of the connection line 5 is shown at two different operating stages. In particular Figure 3 shows the opening stage of the connection line 5 (consisting of a hose) since the closing element 7 is not acting upon it.

In figure 4, instead, an end 12 of the closing member 7 is coupled by snap to a step 13 that is comprised in the other end of the same closing member, thus causing the mutual approach of two projecting portions 14 and 15 with consequent narrowing, up to complete closure, of the hose 16 of the connection line 5.

A pocket container of this type, comprising two or more separate compartments that can be coupled by breaking of such frangible stoppers, can be used also for other medical uses (for instance phleboclysis) in hospital field.

## Claims

1. Pocket container for haemofiltration solutions and for liquids for medical use in general, comprising a first and a second compartment (2, 3), separate from each other and each one containing a solution that is incompatible with the other, a connection line (5), that allows the communication between said first and second compartment (2, 3), and a hose (8) coming out of said first compartment (2) and suitable to connect said container with the patient under dialysis, a frangible stopper (6) being inserted in said connection line (5), which is able to prevent said first and said second compartments (2, 3), from communicating with each other and the breaking of which, that can be done from the outside of said connection line (5) at the moment of use, allows the solution contained in said second compartment (3) to flow into said first compartment (2), **characterized in that** said connecting line further comprises a closing member (7), that is operable to interrupt the communication between said first and second compartment (2, 3) by tightening said connection line (5). After breaking of said frangible stopper (6).

2. Container according to the claim 1, **characterized in that** said frangible stopper (6) comprises a cylindrical and hollow part (9) in which a solution can flow, and a tabbed end (10) connected with said cylindrical part (9) by means of a hollow frangible part (11), in such a way that after breaking of said frangible part (11) said tabbed end (10) is then separated from said cylindrical and hollow part (9) and the solution can flow through said cylindrical and hollow part (9) and through the spaces of the tabs of said tabbed end (10).

3. Container according to any one of the previous claims, **characterized in that** said first and second compartment (2, 3) comprise coupling elements (4).

4. Container according to any one of the previous claims, **characterized in that** it is used as a pocket for phleboclysis.

5. Container according to any one of the previous claims, **characterized in that** said compartments (2, 3) are joined to each other along a frangible line (17).

6. Container according to any one of the previous claims, **characterized in that** said compartments (2, 3) belong to separate pockets.

## Patentansprüche

1. Behälter mit Taschen für Hämofiltrationsflüssigkeiten und für Flüssigkeiten zur allgemeinen medizinischen Verwendung, mit
einer ersten und einer zweiten Kammer (2, 3), die voneinander getrennt sind und von denen jede eine Flüssigkeit enthält, die mit der anderen nicht kompatibel ist,
einer Verbindungsleitung (5), die eine Verbindung zwischen der ersten und der zweiten Kammer (2, 3) ermöglicht, und
einem Schlauch (8), der aus der ersten Kammer (2) herausführt und geeignet ist, den Behälter mit dem Dialysepatienten zu verbinden;
wobei ein brechbarer Stopfen (6) in die Verbindungsleitung (5) eingesetzt ist, der in der Lage ist, zu verhindern, dass die erste und die zweite Kammer (2, 3) miteinander verbunden sind, und dessen Zerbrechen, das im Augenblick der Verwendung von außerhalb der Verbindungsleitung (5) durchgeführt werden kann, es der in der zweiten Kammer (3) enthaltenen Flüssigkeit ermöglicht, in die erste Kammer (2) zu fließen,
**dadurch gekennzeichnet, dass**
die Verbindungsleitung weiter ein Verschlusselement (7) aufweist, das so betrieben werden kann, dass es nach dem Zerbrechen des brechbaren Stopfens (6) die Verbindung zwischen der ersten und der zweiten Kammer (2, 3) durch Zusammendrücken der Verbindungsleitung (5) unterbricht.

2. Behälter nach Anspruch 1, **dadurch gekennzeichnet, dass** der brechbare Stopfen (6) einen zylindrischen und hohlen Abschnitt (9), indem eine Flüssigkeit fließen kann, und ein Ende mit Vorsprüngen (10) aufweist, das durch einen hohlen brechbaren Abschnitt (11) mit dem zylindrischen Abschnitt (9) verbunden ist, so dass das Ende mit Vorsprüngen (10) nach dem Zerbrechen des brechbaren Abschnitts (11) von dem zylindrischen und hohlen Abschnitt (9) getrennt ist und die Flüssigkeit durch den zylindrischen und hohlen Abschnitt (9) und durch die Zwischenräume zwischen den Vorsprüngen des Endes mit Vorsprüngen (10) fließen kann.

3. Behälter nach einem der vorstehenden Ansprüche, **dadurch gekennzeichnet, dass** die erste und die zweite Kammer (2, 3) Koppelelemente (4) aufweisen.

4. Behälter nach einem der vorstehenden Ansprüche, **dadurch gekennzeichnet, dass** er als Tasche für Phleboklysis verwendet wird.

5. Behälter nach einem der vorstehenden Ansprüche, **dadurch gekennzeichnet, dass** die Kammern (2, 3) über eine brechbare Leitung (17) miteinander verbunden sind.

6. Behälter nach einem der vorstehenden Ansprüche, **dadurch gekennzeichnet, dass** die Kammern (2, 3) zu verschiedenen Taschen gehören.

## Revendications

1. Poche pour des solutions d'hémofiltration et pour des liquides pour une utilisation médicale en général, comportant un premier et un deuxième compartiment (2, 3) séparés l'un de l'autre et contenant chacun une solution qui est incompatible avec l'autre, une conduite de raccordement (5), qui permet la communication entre lesdits premier et deuxième compartiments (2, 3), et un tuyau (8) sortant dudit premier compartiment (2) et prévu pour relier ladite poche au patient en cours de dialyse, un élément d'arrêt pouvant être cassé (6) étant inséré dans ladite conduite de raccordement (5), qui est capable d'empêcher lesdits premier et deuxième compartiments (2, 3) de communiquer l'un avec l'autre, et dont la rupture, qui peut être faite de l'extérieur de ladite conduite de raccordement (5) au moment de l'utilisation, permet à la solution contenue dans ledit deuxième compartiment (3) de s'écouler dans ledit premier compartiment (2), **caractérisée en ce que** ladite conduite de raccordement comporte en outre un élément de fermeture (7), qui peut agir afin d'interrompre la communication entre lesdits premier et deuxième compartiments (2, 3) en serrant ladite conduite de raccordement (5) après rupture dudit élément d'arrêt pouvant être cassé (6).

2. Poche selon la revendication 1, **caractérisée en ce que** ledit élément d'arrêt pouvant être cassé (6) comporte une partie cylindrique creuse (9) dans laquelle une solution peut s'écouler, et une extrémité à patte (10) reliée à ladite partie cylindrique (9) au moyen d'une partie creuse pouvant être cassée (11), d'une manière telle que, après rupture de ladite partie pouvant être cassée (11), ladite extrémité à patte (10) est alors séparée de ladite partie cylindrique creuse (9) et la solution peut s'écouler à travers ladite partie cylindrique creuse (9) et à travers les espaces des pattes de ladite extrémité à patte (10).

3. Poche selon l'une quelconque des revendications précédentes, **caractérisée en ce que** lesdits premier et deuxième compartiments (2, 3) comportent des éléments de raccordement (4).

4. Poche selon l'une quelconque des revendications précédentes, **caractérisée en ce qu'**elle est utilisée en tant que poche pour phléboclyse.

5. Poche selon l'une quelconque des revendications précédentes, **caractérisée en ce que** lesdits compartiments (2, 3) sont reliés l'un à l'autre le long d'une conduite pouvant être cassée (17).

6. Poche selon l'une quelconque des revendications précédentes, **caractérisée en ce que** lesdits compartiments (2, 3) appartiennent à des poches séparées.
